# EUROPEAN PATENT SPECIFICATION

(11) **EP 0 484 961 B1**
(45) Date of publication and mention of the grant of the patent: **13.08.1997**
(21) Application number: 91119059.3
(22) Date of filing: 08.11.1991
(51) Int. Cl.: G01N 33/74, G01N 33/68, G01N 33/577, C07K 14/00

(54) **Method of measuring human c-peptide**
Verfahren zur Messung von menschlichem C-Peptid
Méthode de mesurer de C-peptide humaine

(30) Priority: 09.11.1990 JP 302679/90
(43) Date of publication of application: 13.05.1992
(73) Proprietor: Tosoh Corporation, Shinnanyo-shi, Yamaguchi-ken, 746 (JP)
(72) Inventor: Hara, Tadashi, Ebina-shi, Kanagawa-ken (JP); Kondo, Masahide, Ebina-shi, Kanagawa-ken (JP); Kyoden, Takahiro, Usa-shi, Ohita-ken (JP)
(74) Representative: VOSSIUS & PARTNER

(56) References cited:
- EP-A- 0 227 351
- ACTA ACADEMIAE MEDICINAE SINICAE, vol. 11, no. 1, February 1989, CHINA; C. WU et al., p. 51
- DIABETES RESEARCH AND CLINICAL PRACTICE, vol. SUPPL, no. 1, 1985, Washington, DC (US); L. ANGELO et al., pp. 18-19
- ENDOCRINOLOGY, vol. 113, no. 6, June 1986, Washington, DC (US); O.D. MADSEN et al., pp. 2135-2144

## Description

This invention relates to a method of measuring human C-peptide by utilizing antibodies specifically recognizing human C-peptide.

Human C-peptide is a peptide composed of 31 amino acid units, which is produced by degradation of proinsulin during the process of biosynthesis of insulin. Human C-peptide is present in an amount equimolar to insulin in pancreatic β-cells, and is secreted into blood according to a stimulus. Accordingly, there is an interrelation between the secretion of active states of insulin and C-peptide. Therefore, by measuring human C-peptide in blood or urine, insulin-dependent diabetes mellitus, insulin-independent diabetes mellitus, insulinoma and insulin autoimmune diseases can be diagnosed. Furthermore, by measuring insulin in blood, the reliability of the diagnosis can be enhanced.

As the methods of measuring C-peptide, there are known the radioimmunoassay utilizing a competitive method using polyclonal antibodies (see Japanese Unexamined Patent Publications No. 52-25767, No. 57-37586 and No. 62-132172), and the enzyme immunoassay utilizing a competitive method using polyclonal antibodies (see Japanese Unexamined Patent Publication No. 1- 165962). Wu, C. et al., Acta Academiae Medicinae Sinicae (1989) 11, 51 disclose a radioimmunoassay for the determination of human C-peptide using monoclonal antibodies which recognize either the N- or C-terminal of human C-peptide.

In the known radioimmunoassay, since a radioactive isotope is used, problems arise with respect to the safety of an operator and disposal of the isotope and other material, and arise further in that a special equipment and a special measuring device are required with regard to the use of the radioactive substance.

Furthermore, the use of polyclonal antibodies has problems in that it is not easy to prepare serums having constant and uniform performances and the maintenance of quality of the polyclonal antibodies is troublesome.

The technical problem underlying the present invention is to solve the foregoing problems involved in the conventional techniques and provide a method of measuring human C-peptide whereby human C-peptide can be measured with a high precision by a simple operation.

The solution to this technical problem is achieved by providing the embodiments characterized in the claims.

Thus, in accordance with the present invention, there is provided a method of measuring human C-peptide which comprises the steps of (a) contacting a sample containing human C-peptide with a first antibody specifically recognizing human C-peptide and a second antibody specifically recognizing human C-peptide at a site thereof different from the site recognized by the first antibody, (b) separating the thus-produced immunoreaction product from the unreacted antibodies, and (c) determining the immunoreaction product or the unreacted antibodies.

The present invention will now be described in detail.

The method of the present invention, i.e., the sandwich method, is described in the following. In this method, monoclonal antibodies or polyclonal antibodies can be used for the immunoassay. The monoclonal antibodies as used can be prepared by a known process (see,for example, G. Kohler, C. Milstein et al; Nature, 256, 495, 1975). The monoclonal antibodies used in the examples given hereinafter are prepared according to this process. The polyclonal antibodies as used can be an antiserum to human C-peptide or a mixture of monoclonal antibodies.

Preferably, the first antibody specifically recognizing human C-peptide is fixed to a solid phase to facilitate the separation of the immunoreaction product from the unreacted antibodies after the immunoreaction. A known method can be adopted for fixing the first antibody to a solid phase. As the solid phase, there can be mentioned, for example, polystyrene, polyethylene, polyvinyl chloride, polycarbonate, cephalose particles, latexes, agarose, cellulose and polymethacrylates.

Preferably, the second antibody specifically recognizing human C-peptide is labelled to facilitate detection of the immunoreaction product or the unreacted second antibody after the immunoreaction. The method of labelling the second antibody and the method of detecting the immunoreaction product or the unreacted second antibody are not particularly limited, and the labelling and detection can be conducted by known methods. The labelling substance that can be used in the present invention is not particularly limited. As the labelling substance, there can be mentioned, for example, enzymes such as peroxidase, β-D-galactosidase, alkaline phosphatase, urease, catalase and β-glucuronidase; radioactive substances such as ³H, ¹²⁵H and ¹³¹I; and fluorescent substances such as fluorescamine, fluorescein isothiocyanate and tetrarhodamine isothiocyanate (TRITC).

When the second antibody is not labelled, it is preferable to use a labelled third antibody specifically recognizing the immunoreaction product or the second antibody to facilitate the detection of the immunoreaction product or the unreacted antibodies.

When the sandwich method is carried out by using the above-mentioned reagents, the order of the contact of the reagents with the sample is not particularly limited. The sequential contact method can be adopted wherein the reagents are sequentially contacted with the sample, or the immunoreaction can be performed by adding the reagents and the sample at the same time. When the second antibody is not labelled and the labelled third antibody is used, the labelled third antibody may be incorporated either in a reaction mixture of the sample, the first antibody and the second antibody in the step (a), or, in the immunoreaction product or the unreacted antibodies after the immunoreaction product is separated from the unreacted antibodies in the step (b). Finally, the immunoreaction product, the unreacted second antibody or the labelled third antibody is detected.

In addition to the above-mentioned substances, conventional reagents such as a substrate, a buffer agent, a rinsing agent and a reaction stopper can be used for the measurement in the sandwich method.

According to the present invention, a plurality of samples can be measured at a high sensitivity and a high safety in a short time by a simple operation as compared with the conventional measuring methods. The measurement of human C-peptide in a sample can be advantageously carried out at a concentration of 0.1 to 50 ng/ml.

The present invention will now be described in detail with reference to the following examples that by no means limit the scope of the invention.

### Example 1

### Measurement of Human C-Peptide with Use of Two Kinds of Monoclonal Antibodies

### (A) Fixation of anti-human C-peptide antibody

In each well of an untreated microtiter plate (96 wells, Nunk® plate supplied by Intermed Co.) was charged with 100 µl of a solution prepared by dissolving 10 µg/ml of mouse-derived anti-human C-peptide antibody (hereinafter referred to as "first antibody") in 0.1M sodium carbonate buffer having a pH value of 9.6, and incubation was carried out at 4°C overnight.

Then the solution was removed from each well and the plate was rinsed three times with a solution (hereinafter referred to as "PBS-T") prepared by dissolving 0.04% by weight of Tween®-20 (surfactant) in a phosphoric acid-buffered physiological saline solution having a pH value of 7.2 (0.01% by weight of phosphoric acid buffer containing 0.85% by weight of NaCl; hereinafter referred to as "PBS"). Then 300 µl of PBS-T solution containing 1.0% by weight of bovine serum albumin (hereinafter referred to as "BSA") was charged in each well, and the blocking treatment was carried out at 4°C and the anti-human C-peptide antibody-fixed microtiter plate was stored in this state.

### (B) Preparation of antibody labelled with alkaline phosphatase (hereinafter referred to as "AP")

A solution of N-succinimidyl-3-(2'-pyridyldithio)-propionate in ethanol was added to a solution of AP (5 mg/ml) in PBS, the mixture was maintained at room temperature for one hour to effect a reaction, and the reaction mixture was dialyzed with PBS.

Separately, a solution of S-acetylmercaptosuccinic anhydride in 1,4-dioxane was added to a solution of 1 mg of a mouse-derived anti-human C-peptide monoclonal antibody (recognizing human C-peptide at a site thereof different from that recognized by the first antibody) in PBS, the mixture was maintained at 30°C for one hour to effect a reaction, and the reaction mixture was dialyzed with PBS.

Then the AP solution and the solution of the mouse-derived anti-human C-peptide monoclonal antibody were mixed together, a 1M solution of hydroxylamine was added to the mixture, and the resultant mixture was allowed to stand at 4°C overnight. An AP-labelled anti-human C-peptide monoclonal antibody was refined from the thus-obtained reaction solution by the high speed liquid chromatography using TSK-Gel G-3000SW (trademark; supplied by Tosoh Corp.).

### (C) Determination of C-peptide in sample

The temperature of the anti-human C-peptide antibody-fixed microtiter plate prepared by the method described in (A) of this example was returned to room temperature and the plate was rinsed with the PBS-T solution, and 50 µl of a standard sample containing human C-peptide at a concentration of 0 to 50 ng/ml was incorporated in each well. The AP-labelled anti-human C-peptide monoclonal antibody prepared by the method described in (B) of this example was diluted with the PBS-T, and 50 µl of the dilution was incorporated in the well. Incubation was carried out in this state at room temperature for 2 hours, and the solution was removed and rinsing with the PBS-T solution was conducted three times. Then 100 µl of a substrate solution composed of 3 mg/ml of p-nitrophenyl phosphate and a 50 mM of a carbonic acid buffer containing 10 mM of magnesium chloride and having a pH value of 9.5 was incorporated in each well. An enzyme reaction is effected at room temperature for 30 minutes and then 100 µl of a 1N sodium hydroxide solution was added to the reaction mixture to stop the enzyme reaction.

The determination of human C-peptide was conducted with respect to each well of the microtiter plate by measuring the absorbance intensity at a wavelength of 405 nm and a reference wavelength of 492 nm by using an automatic titer plate reader (Model MPR-A4 supplied by Tosoh Corp.). The results are shown in Table 1.

**TABLE 1**

| Concentration of C-peptide (ng/ml) | Absorbance intensity |
|---|---|
| 0 | 0.01 |
| 0.1 | 0.05 |
| 0.3 | 0.24 |
| 1.0 | 0.93 |
| 3.0 | 1.55 |
| 10 | 2.10 |
| 30 | 2.46 |
| 50 | 2.60 |

### Example 2

### Measurement of Human C-Peptide Using Polyclonal Antibody and Monoclonal Antibody

The procedures described in Example 1 were repeated wherein a solution of anti-human C-peptide antiserum derived from a rabbit was used as the fixation antibody instead of the anti-human C-peptide antibody used in the method described in (A) of Example 1, with all other conditions remaining substantialy the same. The results of the human C-peptide determination are shown in Table 2.

**TABLE 2**

| Concentration of C-peptide (ng/ml) | Absorbance intensity |
|---|---|
| 0 | 0.01 |
| 0.1 | 0.12 |
| 0.3 | 0.34 |
| 1.0 | 0.80 |
| 3.0 | 1.17 |
| 10 | 1.30 |
| 30 | 1.49 |
| 50 | 1.59 |

## Claims

1. A method of measuring human C-peptide which comprises the steps of (a) contacting a sample containing human C-peptide with a first antibody specifically recognizing human C-peptide and a second antibody specifically recognizing human C-peptide at a site thereof different from the site recognized by the first antibody, (b) separating the thus-produced immunoreaction product from the unreacted antibodies, and (c) determining the immunoreaction product or the unreacted antibodies.

2. A method as claimed in claim 1 wherein the first antibody is fixed to a solid phase.

3. A method as claimed in claim 1 or 2 wherein the second antibody is labelled with a labelling substance selected from the group consisting of enzymes, radioactive substances and fluorescent substances.

4. A method as claimed in claim 1 or 2 wherein the second antibody is not labelled; and a labelled third antibody specifically recognizing the second antibody is incorporated either in a reaction mixture of the sample, the first antibody and the second antibody in the step (a), or, in the immunoreaction product or the unreacted antibodies after the immunoreaction product is separated from the unreacted antibodies in the step (b).

## Patentansprüche

1. Verfahren zur Messung von menschlichem C-Peptid, umfassend die Schritte: (a) Inkontaktbringen einer Probe, die menschliches C-Peptid enthält, mit einem ersten Antikörper, der spezifisch menschliches C-Peptid erkennt, und einem zweiten Antikörper, der spezifisch menschliches C-Peptid an einer Stelle erkennt, die verschieden von der Stelle ist, die vom ersten Antikörper erkannt wird, (b) Abtrennung des so produzierten Immunreaktionsprodukts von nicht umgesetzten Antikörpern, und (c) Bestimmung des Immunreaktionsprodukts oder der nicht umgesetzten Antikörper.

2. Verfahren nach Anspruch 1, wobei der erste Antikörper an einer festen Phase fixiert ist.

3. Verfahren nach Anspruch 1 oder 2, wobei der zweite Antikörper markiert ist mit einer Markierungssubstanz, ausgewählt aus Enzymen, radioaktiven Substanzen und fluoreszierenden Substanzen.

4. Verfahren nach Anspruch 1 oder 2, wobei der zweite Antikörper nicht markiert ist; und ein markierter dritter Antikörper, der spezifisch den zweiten Antikörper erkennt, zusammengebracht wird entweder mit dem Reaktionsgemisch aus der Probe, dem ersten Antikörper und dem zweiten Antikörper in Schritt (a), oder mit dem Immunreaktionsprodukt oder den nicht umgesetzten Antikörpern, nachdem das Immunreaktionsprodukt von den nicht umgesetzten Antikörpern in Schritt (b) abgetrennt worden ist.

## Revendications

1. Procédé de mesure du peptide C humain qui comprend les étapes consistant à : (a) mettre en contact un échantillon contenant un peptide C humain avec un premier anticorps reconnaissant de manière spécifique le peptide C humain et avec un deuxième anticorps reconnaissant de manière spécifique un site du peptide C humain différent du site reconnu par le premier anticorps, (b) séparer le produit de l'immunoréaction ainsi produit des anticorps qui n'ont pas réagi , et (c) déterminer le produit de l'immunoréaction ou les anticorps qui n'ont pas réagi .

2. Procédé selon la revendication 1, dans lequel le premier anticorps est fixé à une phase solide.

3. Procédé selon la revendication 1 ou 2 dans lequel le deuxième anticorps est marqué avec une substance marqueur choisie parmi des enzymes, des substances radioactives et des substances fluorescentes.

4. Procédé selon la revendication 1 ou 2 dans lequel le deuxième anticorps n'est pas marqué, et dans lequel un troisième anticorps marqué reconnaissant de manière spécifique le deuxième anticorps est incorporé, soit au mélange réactionnel contenant l'échantillon, le premier et le deuxième anticorps de l'étape (a) , soit au produit de l'immunoréaction, soit aux anticorps qui n'ont pas réagi , après avoir séparé le produit de l'immunoréaction des anticorps qui n'ont pas réagi dans l'étape (b).
